(19) Europäisches Patentamt / European Patent Office / Office européen des brevets

(11) **EP 3 442 606 B2**

(12) **NEW EUROPEAN PATENT SPECIFICATION**
After opposition procedure

(45) Date of publication and mention of the opposition decision:
**05.07.2023 Bulletin 2023/27**

(45) Mention of the grant of the patent:
**11.03.2020 Bulletin 2020/11**

(21) Application number: **17716456.3**

(22) Date of filing: **07.04.2017**

(51) International Patent Classification (IPC):
***A61L 15/58*** *(2006.01)* ***A61L 24/04*** *(2006.01)*
***A61L 24/06*** *(2006.01)*

(52) Cooperative Patent Classification (CPC):
(C-Sets available)
**A61L 24/046; A61L 15/58; A61L 24/06;**
A61L 2400/14 (Cont.)

(86) International application number:
**PCT/DK2017/050112**

(87) International publication number:
**WO 2017/178028 (19.10.2017 Gazette 2017/42)**

(54) **METHOD FOR APPLYING AN ADHESIVE**

VERFAHREN ZUM AUFBRINGEN EINES KLEBSTOFFS

PROCÉDÉ D’APPLICATION D’UN ADHÉSIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.04.2016 DK 201670226**

(43) Date of publication of application:
**20.02.2019 Bulletin 2019/08**

(73) Proprietor: **Coloplast A/S**
**3050 Humlebaek (DK)**

(72) Inventors:
- **HANSEN, Kristoffer**
  **2760 Maaloev (DK)**
- **BEJENARIU, Anca Gabriela**
  **2300 Copenhagen S (DK)**
- **OVERGAARD, Anne Kathrine Kattenhoej Sloth**
  **2830 Virum (DK)**
- **STROEBECH, Esben**
  **2970 Hoersholm (DK)**
- **LAM, Peter Kwok Hing**
  **2000 Frederiksberg C (DK)**
- **SUND, Anders Grove**
  **2870 Dyssegaard (DK)**

(56) References cited:
**EP-A1- 2 371 920**
**WO-A1-90/13420**
**WO-A1-99/29273**
**WO-A1-2004/108175**
**WO-A1-2014/093246**
**WO-A1-2015/132551**
**WO-A1-2016/055075**
**WO-A1-2016/124202**
**WO-A1-2016/124203**
**WO-A2-2012/048128**
**US-A1- 2010 255 239**
**US-A1- 2013 089 581**

- **SANTINI et al.: "PHOTOINITIATORS IN DENTISTRY: A REVIEW", Primary Dental Journal, vol. 2, 4 October 2013 (2013-10-04), DOI: 10.1308/205016814809859563**
- **NEUMAN et al.: "The effect of the mixtures of photoinitiators in polymerization efficiencies", Journal of Applied Polymer Science, vol. 112, no. 1 , pages 129-134,**
- **CZECH et al.: "UV-CROSSLINKABLE ACRYLIC PRESSURE-SENSITIVE ADHESIVES FOR INDUSTRIAL APPLICATION", Polym. Bull, vol. 69 4 March 2012 (2012-03-04), pages 71-80, DOI: 10.1007/s00289-012-0725-y**

EP 3 442 606 B2

(52) Cooperative Patent Classification (CPC): (Cont.)

C-Sets
**A61L 15/58, C08L 33/08, C08L 75/04;**
**A61L 24/046, C08L 75/04;**
**A61L 24/06, C08L 33/08**

## Description

The scope of the invention is defined by the claims. Any references in the description to methods of treatment by therapy refer to the compositions for use in a method for treatment of the human (or animal) body by therapy. The invention relates to methods for applying an adhesive to the skin of a user, and to methods for controlling the properties of a skin adhesive, for instance in order to ensure proper and easy application to and removal from the skin.

WO2012048128, WO9013420 and WO2014093246 disclose switchable and reversible adhesive compositions.

## Detailed Description

Embodiments provide a non-therapeutic method for applying a light-switchable adhesive composition to the skin of a user and for removing the light-switchable adhesive composition from the skin surface, wherein the light-switchable adhesive composition comprises at least two different photoinitiators with different absorption spectra the method comprising the steps of

- providing an adhesive composition comprising a switchable adhesive,
- applying the adhesive composition to the skin of the user,
- exposing the switchable adhesive to a first switch activator, thereby switching the switchable adhesive from an application state to a wear state,
- exposing the switchable adhesive to a second switch activator, thereby switching the switchable adhesive from the wear state to a removal state,
- removing the adhesive composition from the skin of the user, wherein the first switch activator is light and wherein the second switch activator is light.

The method may be carried out by a person other than the user to which the adhesive is attached. For instance, the method may be carried out by a commercial service provider assisting the user for a fee. Such commercial service providers exist and provide fee-based services to, e.g., ostomy users or people with wounds. The services may consist of the service provider removing and applying ostomy bags for the ostomy user or removing and applying wound dressings for a person with wounds. The method may also be carried out in order to obtain a sample of the output from the ostomy user or wound exudate from the person with wounds. For instance, a healthcare professional may require a stoma output or wound exudate sample in order to make medical decisions or generally assess the physical state of a user. In such cases, the healthcare professional may order the sampling to be done by a professional service provider to ensure that the sampling happens correctly. Again, a fee-based commercial service provider would carry out the method with the aim of providing a sample to the healthcare professional. Such paid services exist on commercial terms and operate on a continuous and independent basis with the aim of financial gain. They are not exclusively dependent for their operation on the instructions of the user in question. For instance, they may work directly under the instructions of a healthcare professional.

One of the main concerns of people using ostomy appliances is that the ostomy adhesive attachment may be compromised resulting in leakage or even complete detachment of the ostomy appliance. Leakage is problematic not only in that it negatively affects the life quality of the ostomy device user but also because it will lead to skin problems. It is difficult to properly attach an adhesive to damaged skin, thus increasing the risk of further leakage and additional skin damage. There exists a need to further reduce the risk of leakage of ostomy devices.

A central challenge in the design of ostomy devices is that the device has to be attached to the skin of the ostomy device user. The skin is not an easy substrate for adhesion: It has a very large and highly irregular surface, it is often moist, and it stretches, bends, and moves as the ostomy user moves about. Also, many ostomy users have scar tissue in the area around the stoma.

Viewed in isolation, adhesion to the skin may be achieved in a number of ways. However, in the design of an adhesive suitable for use in an ostomy device, several other requirements should be considered. The ostomy adhesive should preferably be able to cope with the moisture evaporating from the skin underneath the ostomy adhesive. The ostomy adhesive should be able to stick to a moist surface, such as moist or sweaty skin, and should, after attachment, be able to somehow reduce accumulation of moisture at the skin surface. Accumulation of moisture at the skin surface can cause maceration of the skin, which is painful and which makes proper adhesion even more difficult to achieve. At the same time, it is of course preferable that the moisture and/or output from inside the collecting bag does not damage the adhesive or leak out to the surface of the ostomy user's skin.

In addition to adhering to the skin and handling moisture, an ostomy adhesive should also be able to remain attached to the skin while carrying a load, namely the collecting bag and its contents. Finally, ostomy adhesives should be able to be removed from the skin, while causing as little damage to the skin as possible and without disintegrating and/or leaving residue on the skin.

The present inventors have provided a novel method for attachment and removal of an adhesive, such as an ostomy adhesive. The method involves providing an adhesive composition that can be switched from an initial application state to a wear state and from the wear state to a removal state. The adhesive composition is applied to the skin of a user in the application state. It is then switched to the wear state and worn by the user for however long the user would normally wear his or her adhesive. Before removal, the adhesive composition is switched again, this time from the wear state to a removal state. In the removal state, the adhesive composition is removed from the skin. Typically, the adhesive is then discarded.

By providing an adhesive that can exist in three separate states, the present inventors have addressed the problem of providing one single adhesive that properly addresses the many different requirements of a skin adhesive. By having three separate states, it is possible to construct a well-functioning and safe adhesive by avoiding some of the usual compromises that have to be made to the application, wear, and removal state when using regular adhesive without the three-state functionality. In particular, the adhesive is capable of good flow and quick initial adherence to the skin in the application state. This helps in preventing leakage from an ostomy device attached by means of the adhesive. It is capable of remaining securely attached in the wear state. And it is capable of easy removal, without leaving residue on the skin, in the removal state. The wear state can have a peel force so high it cannot be removed without first switching it to the removal state. This can make the adhesive safer to wear and potentially avoid leakage issues. Skin health is also improved by the easy and non-traumatic removal made possible by the switch to the low-peel removal state of the adhesive.

In embodiments, the properties of the adhesive composition are different in the three different states, i.e., the application state, the wear state, and the removal state. For instance, the degree of crosslinking may increase from the application state to the wear state and then further increase from the wear state to the removal state. In embodiments, the complex viscosity, measured as described herein, is higher in the wear state than in the application state and/or higher in the removal state than in the wear state. In embodiments, the peel force, measured as described herein, is lower in the application state than in the wear state and/or higher in the wear state than in the removal state. In embodiments, the peel force is higher in the removal state than in the application state. In embodiments, the adhesive composition in the application state exhibits cohesive failure when peel tested as described herein. In embodiments, the adhesive composition in the wear state and/or in the removal state exhibits adhesive failure when peel tested as described herein. In embodiments, the properties of the adhesive are not identical in the application state and in the removal state.

In embodiments, the switch from application state to wear state is irreversible. In embodiments, the switch from the wear state to the removal state is irreversible. This provides the advantage that no accidental switch back from one state to the previous state is possible. For instance, an irreversible switch from the application state to the wear state will ensure that the adhesive cannot suddenly switch back to the application state, which could be problematic in terms of making sure the adhesive stays securely bonded to the skin of the user. For the user, such irreversible switching would provide an increased feeling of security, which is a key parameter in the quality of life of, e.g., ostomy device and wound dressing users. By an irreversible switch is meant that the switch occurs as a consequence of a chemical and/or physical reaction that cannot be made to go in the opposite direction without destroying the adhesive composition. For instance, chemical crosslinking or polymerization are typically irreversible processes. On the contrary, the process of changing the properties of a material by gently heating it and subsequently cooling it down again will typically be a reversible process.

The switchable adhesive is a light-switchable adhesive. The switch activator is light. A light-switchable adhesive is an adhesive that can be switched from one state to another state by exposure to light. Using light as the switch activator is convenient because light is generally easy to apply and can be applied with existing and easy-to-use technology, such as small lamps and flashlights.

The light-switchable adhesive comprises a first switch initiator. The first switch initiator is a first photoinitiator. Using photoinitiators is a preferred way of making the adhesive composition light switchable. Photoinitiators can be built into the adhesive, for instance by covalent bonding to an adhesive polymer, or can be simply added to the composition. It is preferred to use built-in photoinitiators because they do not leach out of the composition during use, thereby making the composition more stable.

The light-switchable adhesive comprises a second switch initiator. The second switch initiator is a second photoinitiator.

According to the invention the light-switchable adhesive comprises at least two different switch initiators. The at least two different switch initiators are two different photoinitiators with different absorption spectra. By providing an adhesive with at least two different switch initiators, it is achieved that the adhesive can be switched, for instance, by sequential activation of the separate switch initiators. The at least two different switch initiators are activated by different switch activators. The adhesive comprises two different photoinitiators with different absorption spectra, meaning that different wavelengths can be used to activate the different photoinitiators. The absorption spectra may be partly overlapping or non-overlapping. By using photoinitiators with different absorption spectra, it is achieved that a certain switch activator, such as a light source with a given spectrum, can be used to activate one of the photoinitiators without activating

the second photoinitiator. Subsequently, a different light source with a different spectrum can be used to activate the second photoinitiator.

In embodiments, the adhesive composition comprises two different photoinitiators with partly overlapping absorption spectra. In embodiments, the exposure to the first switch activator comprises exposing the adhesive to a light of wavelength(s) within the absorption spectrum of the first photoinitiator and outside the absorption spectrum of the second photoinitiator. In embodiments, the exposure to the second switch activator comprises exposing the adhesive to light of wavelength(s) within the absorption spectra of both the first and the second photoinitiator. In this way, the first switch from the application state to the wear state is achieved by activating only the first photoinitiator, thus allowing for an easily controllable and well-defined switch. The second switch, from the wear state to the removal state, is then accomplished by exposing the adhesive to light that activates both of the photoinitiators in the adhesive. This may allow for a more powerful and possibly quicker switch, which can be helpful in making sure the adhesive is switched as much as possible and as quickly as possible to the removal state. In embodiments, the first switch initiator is a photoinitiator that is activated by wavelengths in both the visible and UV portion of the spectrum, and the second switch initiator is a photoinitiator that is activated by light only in the UV spectrum. In this way, the exposure to the first switch activator comprises exposure to visible light, thus activating the first photoinitiator, and the exposure to the second switch activator comprises exposure to UV light, thus activating both photoinitiators.

In embodiments, the first and/or the second and/or the two different photoinitiators are independently selected from the group consisting of α-hydroxyketone, benzophenone, benzophenone derivatives, benzophenone/ α-hydroxyketone, phenylglyoxylate, benzyldimethyl-ketal, aminoketone, acylphosphine derivatives, mono acyl phosphine (MAPO), MAPO/α-hydroxyketone, bis acyl phosphine (BAPO), BAPO dispersion, BAPO/ α-hydroxyketone, phosphine oxide, metallocene, ionium salt, thioxanthone derivatives, mixture of triarylsulphonium hexafluorophosphate salts in propylene carbonate, mixture of triarylsulphonium hexafluoroantimonate salts in propylene carbonate, amphorquinone derivatives, benzil derivatives, anthraquinone derivatives, benzoin ether derivatives, polysilanes, 2-benzyl-2-(dimethylamino)-4'-morpholinobutyrophenone, 2-methyl-4'-(methylthio)-2-morpholinopropiophenone, (benzene) tricarbonylchromium, (cumene)cyclopentadienyliron(II)hexafluorophophate, dibenzosuberenone, ferrocene, methylbenzoylformate, and mixtures thereof.

In embodiments, the light is selected from the group consisting of visible light, ultraviolet (UV) light, and mixtures thereof. Visible light is sometimes preferred due to the ease and safety of using light within the visible part of the spectrum. UV light is sometimes preferred because it is generally easier to control exposure to UV light.

In embodiments, the adhesive composition comprises an adhesive polymer selected from the group consisting of acrylate polymers, acrylate copolymers, and polyurethane polymers.

In embodiments, the adhesive composition in the application state has a complex viscosity $|\eta^*|$ below 0.4 MPa s, measured as described herein. In embodiments, the complex viscosity $|\eta^*|$ in the application state is below 100,000 Pa s, such as below 50,000 Pa s, such as below 20,000 Pa s, such as below 15,000 Pa s. In embodiments, the complex viscosity $|\eta^*|$ in the application state is in the range 1,000 to 50,000 Pa s. Using a low viscosity in the application state is preferred in order for the adhesive to quickly wet and flow into the macro- and micro-structures of the skin.

In embodiments, the adhesive composition in the wear state has a higher complex viscosity than in the application state, measured as described herein. In embodiments, the complex viscosity $|\eta^*|$ in the wear state is above 20,000 Pa s, such as above 30,000 Pa s, such as above 40,000 Pa s, such as above 50,000 Pa s, such as in the range 50,000 to 100,000 Pa s. It is typically preferred to have a relatively high viscosity in the wear state in order for the adhesive to not flow too much during wear.

In embodiments, the adhesive composition in the wear state has a peel force above 1 N/25mm, measured as described herein. A peel force above 1 N/25mm is preferable in that it helps ensure that the adhesive remains securely attached to the skin during wear. In embodiments, the peel force in the wear state is above 5 N/25mm, such as above 8 N/25mm, such as above 10 N/25mm, such as above 12 N/25mm. In embodiments, the peel force in the wear state is in the range 10-15 N/25mm.

In embodiments, the adhesive composition has a lower peel force in the removal state than in the wear state, measured as described herein. In embodiments, the peel force in the removal state is below 12 N/25mm, such as below 11 N/25mm, such as below 10 N/25mm, such as in the range 5-10 N/25mm. It is preferred to have a relatively low peel force in the removal state in order for the adhesive to be easily removable from the skin.

In embodiments, the adhesive composition further comprises water absorbent material. Providing a water absorbent material is a preferred way of handling moisture on the skin of the user.

In embodiments, the adhesive composition further comprises one or more additional components selected from the group consisting of plasticizers, oils, tackifiers, fillers, and viscosity-modifiers.

In embodiments, the adhesive composition is provided in the form of an adhesive wafer comprising the adhesive composition, a backing layer, and a release liner. In embodiments, the adhesive composition is provided in the form of a wound dressing comprising the adhesive composition. In embodiments, the adhesive composition is provided in the form of an ostomy device comprising the adhesive composition. An ostomy device can be suitable for use in connection

with a colostomy, an ileostomy, or a urostomy. An ostomy device may be a closed appliance.

An ostomy device may be an open appliance. An open ostomy appliance is configured for emptying while the appliance is attached to the skin of the user; typically through a drainage port in the bottom of the bag.

An ostomy device can be a one-piece appliance comprising a) a base plate (also referred to as a body-side member or face plate) attachable around the stomal opening; and comprising b) attached to the base plate a collection bag.

On ostomy device can be a two-piece appliance comprising a) a base plate (also referred to as a body-side member) attachable around the stomal opening; and comprising b) a separate collection bag attachable to the base plate. In this two-piece configuration, the collection bag can be replaced without replacing the base-plate attached to the skin around the stomal opening. The separate collection bag may be attached to the body side member in any convenient manner known per se, e.g., via a mechanical coupling, such as a coupling ring, or by an adhesive flange.

In embodiments, the adhesive composition is for securing the adhesive wafer of a two-piece ostomy device to the skin of a user. It may be advantageous to use the present composition in connection with a two-piece device, since the first switching of the adhesive, from the application to the wear state, can be carried out without the presence of the collecting bag, which may be attached after the adhesive has been switched. This is particularly advantageous for compositions that are switched by application of light.

In embodiments, the composition comprises an acrylate, including methacrylates and their copolymers. Acrylate copolymers are especially preferred, e.g., alkyl acrylate copolymers.

The most commonly used monomers in polyacrylates include ethyl acrylate, butyl acrylate, ethylhexyl acrylate, hydroxyethyl acrylate, lauryl acrylate, and acrylic acid. They may be used singly or in a mixture, their relative proportions in the mixture being selected depending on the desired viscoelastic properties, glass transition temperature, compatibility etc.

The polymer may be a copolymer with one or more acrylates. Alternatively, the polymer may be a copolymer with one or more acrylates and a free radical polymerisable vinyl moiety. Such vinyl moieties include compounds such as itaconic anhydride, maleic anhydride or vinyl azlactone or glycidyl methacrylate.

The polymer may be a homopolymer, a random copolymer, or a block copolymer. The polymer may be branched or linear.

The composition may include bound-in curable moieties. Any conventionally known unsaturated compounds, e.g. olefinic or aromatic compounds may be used or compounds with labile groups or groups which can undergo free radical reactions, could be used as the curable molecules. Photoreactive groups may also be used and include groups such as anthracenes, cinnamates, maleimides and coumarin groups. Other functional groups include carboxyl, epoxy, urethane, siloxane, amides, and hydroxyl. Mixtures of all of the above may also be used. The bound-in curable groups may be end groups, pendant groups or may be incorporated into the backbone.

The polymer backbone may be partially crosslinked. Crosslinking can be achieved by incorporating monomers of e.g. N-methylol acrylamide, N-(iso-butoxymethylene)-acrylamide, methyl acrylamidoglycolate methyl ether (all 0.5-5% (w/w)) or metal chelates, e.g., acetylacetonates of Zr, Al, or Fe (up to 2% (w/w) of polymer weight), into the polymer backbone, which then crosslinks during drying after spreading on a substrate. Al and Ti acetylacetonates and similar compounds can also be added after polymerization in concentrations of 0.1-2% (w/w) and used as a crosslinker through utilizing carboxylic groups in the polymer backbone during the drying step.

Multi-functional isocyanates, like toluene diisocyante (TDI), trimethyl hexamethylene diisocyanate (TMDI), and hexamethylene diisocyante (HDI), can be used to chemically link hydroxylic or carboxylic functions of different polymer chains, added in concentration up to 1% (w/w).

Crosslinking can also be achieved between the carboxylic groups in the polymer backbone and added amino resins, such as derivatives of melamine, benzoguanamine, glycoluril, and urea, e.g., hexamethoxymethyl melamine, methoxymethyl methylol melamine, methoxymethyl ethoxymethyl benzoguanamine, tetrabutoxymethyl glycoluril, butoxymethyl methylol urea, in concentrations up to 6% (w/w).

The above-mentioned crosslinking can also be achieved using polycarbodiimides or multifunctional propylene imines.

It is also possible to blend one or more polymers having high cohesive strength with one or more polymers having low cohesive strength in order to achieve the desired balance.

The polymer will most often be soluble in, and hence commercially supplied as solutions in, organic solvents such as ethyl acetate, hexane, toluene, acetone, etc. Preferably, the polymer is non-water-soluble.

The polymer may be a commercially available PSA or PSA precursor, e.g. acResin A 204 UV, acResin A 260 UV (BASF), Aroset 1450-Z-40, Aroset S390 (Ashland), GMS 788, GMS 1753 (Henkel).

The polymer may include curable molecules, which may be low molecular weight monomers or oligomers. In the broadest sense, any conventional known unsaturated compounds, or compounds with labile groups or groups, which can undergo free radical reactions, could be used as the curable molecules. Preferred examples, used alone or in mixtures, are curable molecules such as acrylic acid esters or methacrylic acid esters of alcohols, glycols, pentaerythritol, trimethylpropane, glycerol, aliphatic epoxides, aromatic epoxides including bisphenol A epoxides, aliphatic urethanes,

silicones, polyesters and polyethers, as well as ethoxylated or propoxylated species thereof.

The curable molecules can have more than one unsaturated or reactive site. With more than a single functionality, they enable chain extension. With multiple functionalities of three or greater they are able to form crosslinked three-dimensional polymeric networks. Examples include CN925 (Arkema), Ebecryl 870 (Allnex).

Preferably, the curable molecules and the polymer are soluble in each other when in the dry state, i.e., in the absence of a solvent. Alternatively, in the case that the polymer and the curable molecules are not mutually soluble in each other when dry, or are only partly mutually soluble, they are uniformly dispersed in the composition.

In embodiments, the composition comprises a polyurethane (PU). Polyurethanes are most commonly obtained by reacting molecules containing two or a higher number of alcohol functionalities with di- or polyisocyanates.

Among the organic diisocyanates that can be used in the synthesis of polyurethanes are: cyloaliphatic isocyanates such as 4,4'-Methylenebis(cyclohexyl isocyanate) (HMDI) and isophore diisocyanate, aromatic isocyanates such as tolylene diisocyanate and 4,4'-diphenyl methyl diisocyanate (MDI) as well as aliphatic isocyanates such as 1,6-hexane diisocyanate. Depending on the type of isocyanate used in the synthesis, different materials properties can be obtained. For example, the use of aromatic isocyanates leads to stiffer polymers with higher melting temperature. HMDI based hard segments are not very crystallisable; whereas MDI based hard segments will readily crystallize under favourable conditions. The symmetry of the hard segment is also relevant. Increasing symmetry favours the crystallization of the hard segment and increases the degree of phase separation, modulus, hardness, etc.

Among diols or polyols that can be employed in the synthesis of polyurethanes in the invention are: polydimethylsiloxane (PDMS) based polyols, such as bis(hydroxyalkyl) terminated PDMS, preferably with a weight averaged molecular weight ($M_w$) between 1,000-6,000 g/mol.

4-{3-[bis(2-hydroxyethyl)amino]propoxy}-1-chloro-9H-thioxanthen-9-one (Thioxanthone diol, depicted below) may be used in the synthesis of polyurethanes to impart a light induced crosslinking ability to the polymers.

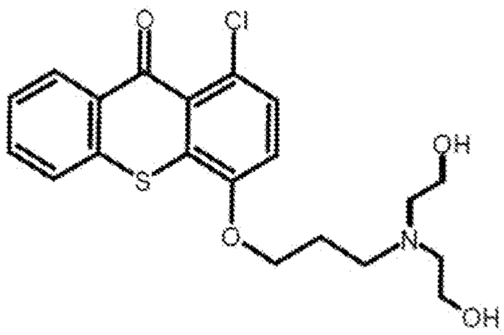

The thioxanthone diol is photo-reactive. In other words, it has the ability to absorb light and initiate free radical reactions, which will lead to crosslinked polyurethanes. The diol functionality in the photo-reactive group enables covalent bonding of these moieties into the polyurethane backbone. Photo-reactive groups built-in to the polymer have less probability to leach out from the polymeric material compared to the case where they are mixed into the polymeric material. This property may help ensure the biosafety of the polymer for direct skin use. Such built-in polymeric photo-initiators can create crosslinks faster than photoinitiators that are simply mixed into the polymer. The photo-initiator mentioned here absorb light mainly in UV as well as to a small extent also in the visible region.

In addition to polyurethanes with built-in photo-reactive groups, polyurethanes with mixed-in photo-initiators are also of interest. Such materials can be prepared by mixing the photo-initiators such as thioxanthone without diol functionalization either during or after the synthesis of the polymer. In these cases, the photo-reactive moiety will not be part of the polyurethane. However, it will still initiate free radical reactions.

The rate of photo-induced crosslinking reaction and the polymer properties can be tuned by the composition of the polyurethane. The type and concentration of photo-initiator, presence of reactive groups as well as polymer mobility are among parameters, which determine the curing speed. The degree of crosslinking is a relevant parameter determining the final properties of the polymer. As the degree of crosslinking increases, the modulus of the polymer increases and mobility of the polymer decreases. The modulus affects the stickiness of the polymer, while mobility influences among various other properties the transport of molecules in the polymer. In case of ostomy care, transport of water molecules in the polymeric material can be an important factor.

The shift between the different states of the composition is referred to herein as a switch. Thus, the switch is the transition from one state to another state of a switchable composition. The duration of the switch will vary depending on, e.g., the nature of the switch initiator and the method of activation of the switch initiator. Generally, the switch will be a gradual process with a gradual change of physical properties of the material from one state to another state. In some instances, the switch will be very fast and the physical properties will change very quickly, e.g. within seconds, to

those of the second state. In other instances, the switch will be slower and the change in properties will gradually happen over a period of, e.g., several minutes or even hours.

The cohesion of the composition may also change as a consequence of the switch. Typically, the switch will render the composition more cohesive, which will help ensure that an adhesive composition can be peeled from a substrate without leaving residue on the substrate, i.e. by adhesive failure and not cohesive failure. For a composition that is to be attached to the skin, it is highly advantageous that the composition can have a high adhesion to the skin and, at the same time, that it can be removed from the skin without leaving residue on the skin. Generally, the cohesion will increase after both the first and the second switch. In other words, the initial cohesion in the application state is relatively low, the cohesion in the wear state is higher, and the cohesion in the removal state is higher again.

The switch is caused by activation of a *switch initiator,* also referred to as exposure to a *switch activator.* The switch initiator is a component of the switchable composition and may, for instance, comprise or consist of a photoinitiator. The switch activator, is light, such as visible light and/or ultraviolet light. The switch initiator can work by one or more mechanisms, such as by generating free radicals. Thus, the switch initiator may be a free radical generating switch initiator. The free radical generating switch initiator may be a photoinitiator.

The activation of the switch initiator, such as activation of a photoinitiator, can lead to increased crosslinking of the polymer and/or increased molecular weight of the polymer.

In embodiments, the switch from the application state to the wear state is irreversible. In this context, irreversible means that once the composition has been switched to the second adhesive state, it cannot revert from that state back to the first liquid state. In embodiments, the switch is irreversible under normal conditions of use. It can be highly advantageous to have an irreversible switch because there is then no risk that the composition will "switch back" to the low-viscous application state when it has been switched to the wear or removal state. For the user, it is important to have a feeling of security with the product. This feeling of security could be compromised if the user had a feeling or knew that the composition could, potentially at any time, switch back to the application state.

In embodiments, the switch initiator comprises or consists of a free radical generating switch initiator. The free radical generating switch initiator may be a photoinitiator. Different photoinitiator systems exist. Photoinitiator systems can be (a) low molecular weight single component, (b) low molecular weight multiple component, (c) polymeric single component, or (d) polymeric multi-component. These systems can be built using chemicals named below and/or polymers containing these functionalities.

In the present invention, a photoinitiator is defined as a moiety, which, on absorption of light, generates reactive species (ions or radicals) and initiates one or several chemical reactions or transformation. In some embodiments, a preferred property of the photoinitiator is good overlap between the UV light source spectrum and the photoinitiator absorption spectrum. In some embodiments, a desired property is a minor or no overlap between the photoinitiator absorption spectrum and the intrinsic combined absorption spectrum of the other components in the composition, e.g., the absorption of the polymer matrix or any absorbent materials and fillers in the composition.

In embodiments, the photoinitiator moieties are pendant on the polymer. This means that they are attached to the polymer at points other than at the polymer ends, thus making it possible to attach more than two photoinitiator moieties to a single polymer.

In embodiments, the composition comprises a built-in photoinitiator. In embodiments, the composition comprises a free or mixed-in or non-built-in photoinitiator. In embodiments, the composition does not comprise a free or mixed-in or non-built-in photoinitiator. In embodiments, the composition does not comprise a built-in photoinitiator. Examples of built in photoinitiators include Dymax 1072-M from Dymax Corp. and Rahn Genopol TX-2 from Rahn Corp.

The photoinitiator moieties of the invention may independently be cleavable (Norrish Type I) or non-cleavable (Norrish Type II). Upon excitation, cleavable photoinitiator moieties spontaneously break down into two radicals, at least one of which is reactive enough to abstract a hydrogen atom. Benzoin ethers (including benzil dialkyl ketals), phenyl hydroxyalkyl ketones, and phenyl aminoalkyl ketones are examples of cleavable photoinitiator moieties.

In embodiments, the photoinitiator is efficient in transforming light from the UV or visible light source to reactive radicals, which can abstract hydrogen atoms and other labile atoms from polymers, and hence effect covalent crosslinking. Optionally, amines, thiols and other electron donors can be either covalently linked to a polymeric photoinitiator or added separately or both. The addition of electron donors is not required but may enhance the overall efficiency of cleavable photoinitiators according to a mechanism similar to that described for the non-cleavable photoinitiators below.

In embodiments, the photoinitiator of the invention is non-cleavable (Norrish Type II). Non-cleavable photoinitiators do not break down upon excitation, thus providing fewer possibilities for the leaching of small molecules from the composition. Excited non-cleavable photoinitiators do not break down to radicals upon excitation, but abstract a hydrogen atom from an organic molecule or, more efficiently, abstract an electron from an electron donor (such as an amine or a thiol). The electron transfer produces a radical anion on the photoinitiator and a radical cation on the electron donor. This is followed by proton transfer from the radical cation to the radical anion to produce two uncharged radicals; of these, the radical on the electron donor is sufficiently reactive to abstract a hydrogen atom.

Benzophenones and related ketones such as thioxanthones, xanthones, anthraquinones, fluorenones, dibenzosuberones, benzils, and phenyl ketocoumarins are examples of non-cleavable photoinitiators. Most amines with a C-H bond in α-position to the nitrogen atom and many thiols will work as electron donors. An advantage of using Norrish Type II as opposed to Type I photoinitiators is fewer generated by-products during photoinitiated reactions. As such, benzophenones are widely used. When for example α-hydroxy-alkyl-phenones dissociate in a photoinitiated reaction, two radicals are formed, which can further dissociate and possibly form loosely bound unwanted aromatic by-products. Self-initiating photoinitiator moieties may also be used. Upon UV or visible light excitation, such photoinitiators predominantly cleave by a Norrish type I mechanism and crosslink further without any conventional photoinitiator present, allowing thick layers to be switched. Recently, a new class of β-keto ester based photoinitiators has been introduced.

The switch initiator comprises at least two different types of photoinitiators. The absorbance peaks of the different photoinitiators are at different wavelengths, so the total amount of light absorbed by the system increases. The different photoinitiators may be all cleavable, all non-cleavable, or a mixture of cleavable and non-cleavable. A blend of several photoinitiator moieties may exhibit synergistic properties. In some embodiments, the switch initiator comprises a mix of different photoinitiators, such as two, three, four, or five different photoinitiators.

Examples of photoinitiators absorbing in the 200-400 nm range include α-hydroxyketone, benzophenone, benzophenone derivatives, benzophenone/ α-hydroxyketone, phenylglyoxylate, benzyldimethyl-ketal, aminoketone, acylphosphine oxide derivatives, mono acyl phosphine (MAPO), MAPO/α-Hydroxyketone, bis acyl phosphine (BAPO), BAPO dispersion, BAPO/ α-hydroxyketone, phosphine oxide, metallocene, ionium salt, thioxanthone derivatives, mixture of triarylsulphonium hexafluorophosphate salts in propylene carbonate, mixture of triarylsulphonium hexafluoroantimonate salts in propylene carbonate, camphorquinone derivatives, benzil derivatives, anthraquinone derivatives, benzoin ether derivatives, and polysilanes.

Specific examples of photoinitiators include 2-Benzyl-2-(dimethylamino)-4'-morpholinobutyrophenone, 2-Methyl-4'-(methylthio)-2-morpholinopropiophenone, (Benzene) tricarbonylchromium (Cumene)cyclopentadienyliron(II)hexafluorophophate, dibenzosuberenone, ferrocene, and methylbenzoylformate.

Other examples include aromatic ketones useful in the 200-400 nm range, e.g. acetophenone; camphorquinone + iodonium salt + silane (which may be useful in obtaining efficient photoinitiation in air); peroxides, e.g. benzoyl peroxide; and azo compounds, e.g. 2,20-azobisisobutyronitrile.

In the >400 nm range examples of photoinitiators include carbazole derivatives, metallocene, thioxanthone derivatives, camphorquinone derivatives, benzil derivatives, titanocenes, anthraquinone derivatives, acylphosphine derivatives, keto-coumarins, xanthenic dyes (e.g. erythhrosin B), thioxanthone derivatives (e.g. 2-chlorothioxanthone, 2-isopropylthioxanthone, 2-mercaptothioxanthone, thioxanthone acetic acid derivatives) optionally in combination with amines, and benzophenones optionally in combination with amines.

In embodiments the switch initiator comprises or consist of bis(eta.5-2,4-cylcopentadien-1-yl)-bis(2,6-difluoro-3-(1H-pyrrol-1-yl)-phenyl)titanium (Ciba Irgacure 784).

In some embodiments, the light comprises visible light and/or UV light. Visible light is defined as electromagnetic radiation with a wavelength in the range 400-700 nm. Ultraviolet light is defined as electromagnetic radiation with a wavelength in the range 10-400 nm.

In some embodiments, the exposure to light has a duration of 10-60 seconds.

For instance, the exposure to light may be less than 60 minutes, less than 30 minutes, less than 10 minutes, less than 5 minutes, less than 4 minutes, less than 3 minutes, less than 2 minutes, less than 1 minute, less than 45 seconds, less than 30 seconds, less than 15 seconds, less than 10 second, less than 5 seconds, 1-10 seconds, 10-30 seconds, 10-60 seconds, 30-60 seconds, 1-2 minutes, 2-3 minutes, 3-4 minutes, or 4-5 minutes.

The composition comprises at least two switch initiators, i.e. two photoinitiators. According to the invention the composition comprises two different photoinitiators with different absorption spectra. In embodiments, the composition comprises a visible-light-absorbing photoinitiator and a non-visible-light-absorbing photoinitiator. In embodiments, the composition comprises a UV-absorbing photoinitiator and a non-UV-absorbing photoinitiator. In embodiments, the composition comprises only a single switch initiator (not according the invention). In the case of a single switch initiator, the switch initiator may be activated twice by the same of different switch activators.

In some embodiments, the adhesive composition in one or more of the application, wear, and removal states is adapted to handle moisture.

Adapted to handle moisture means that the composition has skin moisture-handling capability, i.e., the composition is able to ensure that the amount of moisture accumulating on the surface of the skin is kept low. The composition could prevent such accumulation by being capable of absorbing moisture from the surface of the skin. The adhesive could also, or alternatively, be moisture vapor permeable, thereby ensuring that the moisture would permeate through the adhesive and away from the skin. Thus, in some embodiments, "adapted to handle moisture" means that the composition is moisture vapor permeable. In some embodiments, "adapted to handle moisture" means that the composition is absorbent, for instance by comprising a water absorbent material, such as a hydrocolloid. In embodiments, the composition is both absorbent and moisture vapor permeable.

It has been reported that humans for short periods can sweat more than 20,000 $g/m^2/24$ h. Thus, the moisture

handling ability of skin adhesives, e.g. the water absorption capacity and the moisture vapour transmission rate (MVTR) of the adhesive, is relevant to the performance of the adhesive.

For a number of reasons, moisture-handling capability is especially relevant for an adhesive used to attach an ostomy device: The adhesive used to attach the ostomy device is placed on approximately the same area of skin around the stoma every day for weeks, months, or years. Thus, the health of this particular area of skin is very important. An adhesive that properly handles moisture will contribute to the health of the skin by keeping the skin relatively dry and thus preventing macerations and other moisture-related damage to the skin. Furthermore, an excessive accumulation of moisture between the skin and the adhesive will lead to a weakened bond of the adhesive to the skin. This is especially problematic for an ostomy device, which has to be able to carry a load and stay very firmly attached to the skin to prevent leakage.

The moisture handling ability of a composition can be controlled for instance by making the composition capable of absorbing water and/or by making the composition moisture vapor permeable. A composition can at the same time be both absorbent and moisture vapor permeable, for instance by including hydrocolloids or other absorbing materials in a moisture vapor permeable composition.

One way of rendering a composition capable of handling moisture is by including absorbent material in the composition. The absorbent material can for instance be a water-soluble or water swellable material and can be added in an amount sufficient to ensure proper handling of the moisture present at the site of the adhesive.

The water absorbent material is suitably a particulate, solid water absorbent hydrophilic agent, such as a water-soluble or a water swellable (non-water soluble) hydrocolloid. The water soluble or water swellable (non-water soluble) hydrocolloids may suitably be selected from natural or synthetic hydrocolloids, such as guar gum, locust bean gum, pectin, alginates, gelatine, xantan or gum karaya, cellulose derivatives (e.g. salts of carboxymethyl cellulose such as sodium carboxymethyl cellulose, methyl cellulose and hydroxypropyl cellulose), sodium starch glycolate, polyvinylalcohol, polyacrylic acid (e.g. in the form of super absorbent particles SAP), and polyethylene glycol. Suitable hydrocolloids are, e.g., AQ 1045 (a branched water dispersible polyester) from Eastman, Pectin LM 12CG Z or Pectin USP/100 from CP Kelco, Natrosol (hydroxyethyl cellulose, non-ionic, water soluble ethers of cellulose and ethylene oxide) produced by AQUALON, Blanose 9H4XF (carboxymethyl cellulose) available from Hercules, Akucell ® AF 2881 (carboxymethyl cellulose) available from Akzo, AquaSorb® (crosslinked carboxymethyl cellulose) from Aqualon, Sorbalg pH 470 (Calcium alginate) from Danisco Ingredients, Denmark. The hydrocolloids may also be selected from microcolloids (e.g having a particle size less than 20 microns or preferably below 5 or 2 microns).

The absorption capacity of a composition can be measured as defined herein. The water absorption capacity can be measured in each of the states of the composition. The water absorption may be the same or different between the different states of the composition. In some embodiments, the water absorption capacity is higher in the application state than in the wear and removal state. In other embodiments, the water absorption capacity is lower in the application state than in the wear state. A good absorption capacity will make the composition capable of handling moisture on the skin and will thereby prevent accumulation of moisture between the skin and the adhesive and thus help prevent damage to the skin, such as maceration.

As an alternative or a supplement to making the composition capable of handling moisture by adding water absorbent materials, the composition can be capable of handling moisture by being moisture vapor permeable. Acrylate compositions can be moisture vapor permeable and examples are provided herein of acrylate compositions with varying degrees of moisture vapor permeability.

The moisture vapor permeability of a composition can be measured by measuring the moisture vapor transmission rate (MVTR) as described herein. In some embodiments, the composition in the application state and/or in the wear state and/or in the removal state has an MVTR above 500 $g/m^2/24h$ measured as described herein.

In some cases, it is desirable to reduce the amount of absorbing material in a given composition. This can be because a high amount of, for instance, hydrocolloids may lead to the composition becoming too hard and/or too easily eroded. In those cases, a moisture vapor permeable composition is advantageous in that it can render a composition with no absorbing material, or a relatively low amount of absorbing material, capable of handling moisture.

Moisture vapor permeable compositions can contain absorbing materials to supplement the moisture handling effect of the moisture vapor permeable composition as such. A composition comprising absorbing material and at the same time being moisture vapor permeable may be advantageous in that the positive effects of absorption and moisture vapor permeability are combined in one composition.

In embodiments, the composition is adapted to handle moisture by being water absorbent.

In embodiments, the composition comprises a water absorbent material.

In embodiments, the composition comprises a water absorbent material in an amount of 1-60% (w/w) of the composition.

For instance, the composition comprises a water absorbent material in an amount of 1-40% (w/w) or 10-40% (w/w) or 1-20% (w/w) or 20-40% (w/w) or 20-60% (w/w) or 40-60% (w/w) or 25-50% (w/w) of the composition.

In embodiments, the water absorbent material is selected from hydrocolloid, water soluble salt, mono, di- and

oligosaccharides, sugar alcohols, polypeptides, organic acids, inorganic acids, amino acids, amines, urea, super absorbent particles such as polyacrylic acid, glycols such as polyethylene glycol, fumed silica, bentone, bentonite, and mixtures thereof.

In embodiments, the hydrocolloid is selected from guar gum, locust bean gum, pectin, potato starch, alginates, gelatine, xantan or gum karaya, cellulose derivatives, salts of carboxymethyl cellulose, sodium carboxymethyl cellulose, methyl cellulose, hydroxypropyl cellulose, hydroxyethyl cellulose, sodium starch glycolate, polyvinylalcohol, and mixtures thereof. Different absorbent materials will have different properties, such as rate of absorption and absorption capacity. For instance, guar gum or polyacrylic acid may be selected for their ability to maintain a relatively high cohesion of the adhesive composition even after significant absorption. On the other hand, carboxymethyl cellulose and similar compounds can be used to provide fast initial absorption rates and high absorption capacity.

In embodiments, the water soluble salt is selected from NaCl $CaCl_2$, $K_2SO_4$, $NaHCO_3$, $Na_2CO_3$, KCl, NaBr, NaI, KI, $NH_4Cl$, $AlCl_3$, $CH_3COONa$, $CH_3COOK$, HCOONa, HCOOK, and mixtures thereof.

In embodiments, the composition in one or more of the application, wear, and removal states has an absorption measured as described herein of at least 0.1 g/cm$^2$/2h, such as at least 0.2, 0.3, 0.4, 0.5, 1, 1.5, 2, 2.5, or 3 g/cm$^2$/2h.

In some embodiments, the absorption of the composition in application state is higher than the absorption of the composition in the wear state and/or the removal state. It is preferable to have an especially high absorption in the application state in order for the adhesive composition to be able to deal with moisture present on the skin of the user in the area where the adhesive composition is to be adhered.

In some embodiments, the composition in one or more of the application, wear, and removal states is adapted to handle moisture by being moisture vapor permeable.

In some embodiments, the moisture vapor transmission rate (MVTR) of the composition in one or more of the application, wear, and removal states is above 250 g/m$^2$/24h, such as above 500, 750, 1000, 1250, 1500, 2000, 2500, or 3000 g/m$^2$/24h, measured as described herein.

The pressure sensitive adhesive used according to the invention may contain other conventional ingredients for compositions, such as tackifiers, extenders, non-reactive polymers, oils (e.g. polypropyleneoxide, ethyleneoxide-propyleneoxide copolymers, and mineral oil), plasticizers, fillers, surfactants. The adhesive may also comprise pharmaceutically active ingredients. These optional ingredients may be present in the reaction mixture during the cross linking reaction.

In some embodiments, the composition in the application state has a complex viscosity $|\eta^*|$ below 50 kPa s. Our experiments have shown that a pressure sensitive adhesive to be used within ostomy care with a complex viscosity below 50 kPa s will enable the adhesive to wet the peristomal skin of the user very fast - hereby quickly creating a large contact surface.

In embodiments, the composition in the application state has a complex viscosity $|\eta^*|$ below 0.3 MPa s, below 0.25 MPa s, below 0.2 MPa s, below 0.1 MPa s, below 50 kPa s, below 10 kPa s, below 5 kPa s, below 1 kPa s, below 500 Pa s, below 100 Pa s, below 50 Pa s, below 10 Pa s, below 1 Pa s, in the range 0.1 - 0.4 MPa s, in the range 10 - 100 kPa s, in the range 1 - 10 kPa s, in the range 100 - 1,000 Pa s, in the range 10 - 100 Pa s, or in the range 1 - 10 Pa s.

In some embodiments, the composition in the application state has a complex viscosity $|\eta^*|$ of at least 1, 10, 20, or 50 Pa s. Such a minimum viscosity will ensure that the composition remains viscous enough to be easily handled and that any particulate components of the compositions, such as hydrocolloids, can be evenly distributed and do not simply "sink" to the bottom of the composition. This will help ensure stability of the composition and make handling of the composition easier. In embodiments, the composition in the application state has a complex viscosity $|\eta^*|$ of at least 100, 500, 1,000, 2,500, 5,000, or 10,000 Pa s.

In some embodiments, the complex viscosity in the wear state is at least 2 times, such as at least 5 times, such as at least 10 times higher than the complex viscosity in the application state.

Generally, in order to reduce the viscosity of a composition, low molecular weight compounds can be added to the composition, e.g. tackifiers, oils, monomers, oligomers, and plasticizers. Certain polymers, such as polyisobutylene, can be reduced in molecular weight by radiation. Also, generally low molecular weight polymers will be less viscous than higher molecular weight polymers.

Generally, the viscosity of a composition can be increased by choosing high molecular weight polymers or by crosslinking the polymer. Also, fillers, e.g. calcium carbonate, magnesium oxide, fumed silica, and lignin, can be added.

The viscosity is measured as described in detail herein. The measured viscosity correlates with the ability of the composition to flow into and wet a rough surface, such as skin. In particular, the indicated viscosity levels were measured at a frequency of 0.01 Hz, which is within the relevant frequency interval for bonding of adhesives to rough surfaces, such as skin. It has been suggested that a frequency of around 0.1 Hz is relevant for bonding of adhesive tapes to a smooth surface. The herein used slightly lower frequency of 0.01 Hz, corresponding to approximately 0.063 rad/s, reflects the experience of the inventors that an adhesive for a rough surface does not require quite as fast adhesion as a tape on a smooth surface and also needs a little more time to flow. In other words, the frequency of 0.01 Hz was chosen to reflect the somewhat slower process of skin adhesive bonding as compared to bonding to typical smooth

substrates and thereby to get viscosity measurements that are as relevant as possible for an adhesive that is to be applied to the skin. The viscosity measurement therefore provides viscosity values that are relevant for the actual application to the skin of, e.g., an ostomy adhesive.

In embodiments, the adhesive composition in the wear state has a peel force above 1 N/25mm, measured as described herein. In embodiments, the adhesive composition has a lower peel force in the removal state than in the wear state, measured as described herein.

Adjusting the peel force of a composition can be done, for instance, by adjusting the degree of crosslinking of the polymer used in the composition. The peel force can also be affected by adding tackifiers and/or plasticizers to the composition. Increasing the content of tackifiers and plasticizers will lead to an increased peel force. Correspondingly, a lower content of tackifiers and/or plasticizers will lead to a lower peel force. Depending on the exact choice of tackifiers and plasticizers, the force can be adjusted without at the same time significantly affecting the viscosity of the composition. Polymers, which are more or less miscible with the base polymer of the composition, may also be added to control peel force. Hydrocolloids, oils, and various fillers can also be used to adjust the peel force. Generally, hydrocolloids and fillers will tend to reduce the peel force of the composition. The peel force is measured as described herein.

**Measurement methods**

***Dynamic mechanical analysis (DMA) and determination complex viscosity*** $|\eta^*|$

Complex viscosity $|\eta^*|$ was measured as follows by a frequency sweep. The adhesives were pressed into a plate of 1 mm thickness. A round sample of 25 mm in diameter was cut out and placed in a Haake RheoStress 6000 rotational rheometer from Thermo Scientific. The geometry applied was parallel plates 25 mm and the shear stress was fixed at 5556 Pa and a gap size of 0.9-1.05 mm was applied to the sample in the beginning of the measurement to obtain a normal force of approximately 5 N. The measurements were carried out at 32°C. For the complex viscosity $|\eta^*|$ the value measured at a frequency of 0.01 Hz was used. The test was run as a frequency sweep from 100 Hz to 0.01 Hz. Complex viscosity may herein also be referred to simply as "viscosity".

***Peel force***

A sample of 25x100 mm was cut from the prepared sheet composition and a 25x300 mm piece of auxiliary tape was then added on the top of the sample. After 30 minutes of conditioning at 23 °C and 50% relative humidity, the sample was mounted in a tensile testing machine (INSTRON 5564 from Instron) and a 90-degree peel test was carried out from a Teflon substrate at a speed of 304 mm/min. The results are given in N/25 mm.

The peel test was carried out in a climate-controlled room at 23 °C and 50% relative humidity. Peel angle was fixed at 90° and the peel speed was 304 mm/min. Dwell time, meaning the time the sample is rested before testing, was 30 minutes.

The Teflon substrate (2.0 mm PFTE, order no. SPTFE0020INA from RIAS, Roskilde, Denmark) mounted in steel plate was attached to the peel sledge. Adhesive strips were punched out from 0.4 mm thick adhesive sheets in the dimensions 25x100 mm. Auxiliary tape (25 mm width) was mounted on the adhesive with 10 mm overlap. The release liner was lifted in one end to make the overlap with the auxiliary tape. The adhesive was applied to the substrate by using an automatic roll with a load of 2 kg. The average of the mean load was reported as N/25 mm. The failure type, i.e. cohesive ("cf') or adhesive failure ("af"), was observed, recorded, and reported with the peel data.

***Moisture vapour transmission rate***

Moisture vapour transmission rate (MVTR) is measured in grams per square meter ($g/m^2$) over a 24 hours period using an inverted cup method.

A container or cup that was water and water vapour impermeable having an opening of Ø35 mm was used. 20 mL saline water (0.9% NaCl in demineralised water) was placed in the container and the opening was sealed with the test adhesive mounted on a highly permeable polyurethane (PU) backing film (BL9601 foil from Intellicoat). The container was placed into an electrically heated cabinet and the container or cup was placed upside down, such that the water was in contact with the adhesive. The cabinet was maintained at 32°C. The film reference is used in all experiments to control for any variations in testing conditions.

The weight loss of the container was followed as a function of time. The weight loss was due to water transmitted through the adhesive and/or film. This difference was used to calculate the MVTR of the test adhesive film. MVTR was calculated as the weight loss per time divided by the area of the opening in the cup ($g/m^2/24h$).

The MVTR of a material is a linear function of the thickness of the material. Thus, when reporting MVTR to characterize a material, it is important to inform the thickness of the material which MVTR was reported.

Finally, we noted that by using this method, we introduced an error by using a supporting PU film. Utilizing the fact that the adhesive/film laminate was a system of two resistances in series eliminated the error. When the film and the adhesive are homogeneous, the transmission rate may be expressed as:

$$1/P(\text{measured}) = 1/P(\text{film}) + 1/P(\text{adhesive}).$$

Hence, by knowing the film permeability and thickness of the adhesive, it is possible to calculate the true permeability of the adhesive, P(adhesive), using the following expression:

$$P(\text{adhesive}) = d(\text{adhesive})/150\mu m * 1/(1/P(\text{measured}) - 1/P(\text{film})),$$

where d(adhesive) was the actual measured thickness of the adhesive and P(film) was the MVTR of the film without any adhesive on and P(measured) was the actual measured MVTR.

***Moisture absorption***

Samples were prepared by thermoforming to a 0.5 mm thick adhesive film between two release liners. With a punching tool, samples were punched out. Sample size was 25 x 25 mm. The release liners were removed. The samples were glued to an object glass and placed in a beaker with physiological salt water and placed in an incubator at 37 °C.

The sample was weighed at the outset (M(start)) and after 2 hours (M(2 hours). Before weighing, the object glass was dried off with a cloth. For a 25 x 25 mm sample the area was 6.25 $cm^2$ (the surface edges were left out of the area). The moisture absorption may be calculated as: Water absorption after 2 hours = (M(2 hours) - M(start))/6.25 $cm^2$. The result is in the unit $g/cm^2$ per 2 hours.

**Examples**

As an example of the type of composition useful in the methods described herein, the inventors produced the following composition.

**Composition 1:** BASF acResin A 260 UV with 0.5% photoinitiator, 25% tackifier, and 25% mixed hydrocolloids

Materials for Composition 1: BASF acResin A 260 UV (acrylic ester based polymer from BASF), Sylvares TR A25L clear, liquid polyterpene tackifier resin (Arizona Chemical), Irgacure 784 photoinitiator from Ciba (Bis(.eta.5-2,4-cylcopentadien-1-yl)-bis(2,6-difluoro-3-(1H-pyrrol-1-yl)-phenyl) titanium), and hydrocolloid mixture consisting of 10 % (w/w) pectin LM CG (CP Kelco), 20% (w/w) Akucell AF288 (Akzo Nobel), 30 % (w/w) PB gelatine (PB Gelatins), and 40 % (w/w) Guar gum FG-20 (Hercules Corp.).

Recipe for Composition 1: 29.7 g of BASF acResin A 260 UV were mixed with 15 g of Sylvares TR A25L for 3 minutes. Afterwards, 15 g of hydrocolloids mixture and 0.30 g Irgacure 784 were added to the mixture and let mix for 20 minutes. The compositions were prepared using a 60g capacity Brabender blade mixer at 90 °C with 30 rotations per minute.

Composition 1 contains two separate and different photoinitiators, a UV-absorbent photoinitiator in the BASF acResin A 260 UV component, and the separately added Irgacure 784 photoinitiator, which absorbs in both the visible light region and the UV region. The composition further includes tackifier, which is added to control the adhesive properties of the composition, and hydrocolloids, which will allow the composition to absorb water.

The composition can be switched by activation of the Irgacure 784 component, which is sensitive to both UV and visible light, and by activation of the A 260 UV component, which is sensitive to UV-light. For instance, the composition can be switched from the application state to the wear state by exposure to visible light. This will activate the Irgacure 784 component and cause a switch. Subsequently, the composition can be switched from the wear state to the removal state by exposure to UV-light, which will activate both the UV-active A 260 UV component and, to some extent, also the Irgacure 784 component.

***Results***

The viscosity of Composition 1 was measured in the initial "application" state, i.e., before switching the composition, and in the "wear" state, i.e., after the first switch of the composition but before the second switch. The first switch was performed by exposure to visible light. A low viscosity will allow the adhesive to flow relatively easily into the

structures of the skin, including scars, folds, and microstructures. A higher cohesion will ensure that the adhesive composition does not flow easily and therefore can remain securely in place. For this reason, it is preferable that the viscosity is low in the application state and higher in the wear state.

The peel force was measured in the application and wear states, as well as in the final "removal" state, i.e., after both switches. The second switch was performed by exposure to UV-light. For the peel force measurements, the failure mode was recorded. Cohesive failure ("cf') means that the adhesive cannot be pulled off the substrate as a single cohesive unit of material, but that it rather is pulled apart, with some of the adhesive remaining stuck to the substrate. Adhesive failure ("af") means that it is the adhesion to the substrate that fails, meaning that the adhesive is pulled off the substrate while remaining intact as a single cohesive material unit. Generally, adhesive compositions with low cohesion will exhibit cohesive failure while composition with a high cohesion will peel with adhesive failure. For a skin adhesive, it is desirable that the adhesive can be removed in one piece, without leaving some of the adhesive still stuck to the skin. Therefore, it is preferable that the adhesive composition fails adhesively in the removal state.

| | **Application state (nonswitched)** | **Wear state (after 1$^{st}$ switch)** | **Removal state (after 2$^{nd}$ switch)** |
|---|---|---|---|
| **Complex viscosity (Pa s at 0.01 Hz)** | 10,890 | 54,551 | Not measured |
| **Peel (N/25mm)** | 7.92 (cf) | 12.67 (af) | 9.74 (af) |

As seen in the table, Composition 1 exhibits different properties in the three different states. In particular, the composition has a relatively low viscosity in the application state and a much higher viscosity in the wear state. This means that the composition can easily flow into the structure of the skin in the application state and that it can remain securely in place in the wear state. Also, it is seen that Composition 1 fails cohesively when measuring peel force in the application state. This makes sense because the adhesive composition in this state is a relatively low-cohesion, flowable paste. In both the wear state and the removal state, the adhesive composition fails adhesively, signifying a higher cohesion in these states as compared to the application state. Importantly, the peel force is high in the wear state, meaning that the adhesive is tightly adhered to the substrate and much lower in the removal state, thus allowing for easy removal from the substrate.

## Claims

**1.** A non-therapeutic method for applying a light-switchable adhesive composition to the skin of a user and for removing the light-switchable adhesive composition from the skin surface, wherein the light-switchable adhesive composition comprises at least two different photoinitiators with different absorption spectra, the method comprising the steps of

- providing an adhesive composition comprising a switchable adhesive,
- applying the adhesive composition to the skin of the user,
- exposing the switchable adhesive to a first switch activator, thereby switching the switchable adhesive from an application state to a wear state,
- exposing the switchable adhesive to a second switch activator, thereby switching the switchable adhesive from the wear state to a removal state,
- removing the adhesive composition from the skin of the user,

wherein the first switch activator is light and wherein the second switch activator is light.

**2.** The method according to any one of the preceding claims, wherein the two different photoinitiators are independently selected from the group consisting of α-hydroxyketone, benzophenone, benzophenone derivatives, benzophenone/a-hydroxyketone, phenylglyoxylate, benzyldimethyl-ketal, aminoketone, acylphosphine derivatives, mono acyl phosphine (MAPO), MAPO/α-hydroxyketone, bis acyl phosphine (BAPO), BAPO dispersion, BAPO/α-hydroxyketone, phosphine oxide, metallocene, ionium salt, thioxanthone derivatives, mixture of triarylsulphonium hexafluorophosphate salts in propylene carbonate, mixture of triarylsulphonium hexafluoroantimonate salts in propylene carbonate, amphorquinone derivatives, benzil derivatives, anthraquinone derivatives, benzoin ether derivatives, polysilanes, 2-benzyl-2-(dimethylamino)-4'-morpholinobutyrophenone, 2-methyl-4'-(methylthio)-2-morpholinopropiophenone, (benzene) tricarbonylchromium, (cumene)cyclopentadienyliron(II)hexafluorophophate, dibenzosuberenone, ferrocene, methylbenzoylformate, and mixtures thereof.

3. The method according to any one of the preceding claims, wherein the switch activator is light selected from the group consisting of visible light, UV light, and mixtures thereof.

4. The method according to any one of the preceding claims, wherein the adhesive composition comprises an adhesive polymer selected from the group consisting of acrylate polymers, acrylate copolymers, and polyurethane polymers.

5. The method according to any one of the preceding claims, wherein the adhesive composition in the application state has a complex viscosity |η*| below 0.4 MPa s, measured as described herein.

6. The method according to any one of the preceding claims, wherein the adhesive composition in the wear state has a higher complex viscosity than in the application state, measured as described herein.

7. The method according to any one of the preceding claims, wherein the adhesive composition in the wear state has a peel force above 1 N/25mm, measured as described herein.

8. The method according to any one of the preceding claims, wherein the adhesive composition has a lower peel force in the removal state than in the wear state, measured as described herein.

9. The method according to any one of the preceding claims, wherein the adhesive composition further comprises water absorbent material.

10. The method according to any one of the preceding claims, wherein the adhesive composition further comprises one or more additional components selected from the group consisting of plasticizers, oils, tackifiers, fillers, and viscosity-modifiers.

11. The method according to any one of the preceding claims, wherein the adhesive composition is provided in the form of an adhesive wafer comprising the adhesive composition, a backing layer, and a release liner.

12. The method according to any one of the preceding claims, wherein the adhesive composition is provided in the form of an ostomy device comprising the adhesive composition.

**Patentansprüche**

1. Nichttherapeutisches Verfahren zum Aufbringen einer lichtschaltbaren Klebstoffzusammensetzung auf die Haut eines Anwenders und zum Entfernen der lichtschaltbaren Klebstoffzusammensetzung von der Hautoberfläche, wobei die lichtschaltbare Klebstoffzusammensetzung wenigstens zwei verschiedene Photoinitiatoren mit unterschiedlichen Absorptionsspektren umfasst, wobei das Verfahren die Schritte umfasst:

   - Bereitstellen einer Klebstoffzusammensetzung, die einen schaltbaren Klebstoff umfasst,
   - Aufbringen der Klebstoffzusammensetzung auf die Haut des Anwenders,
   - Exponieren des schaltbaren Klebstoffs gegenüber einem ersten Schaltaktivator, dadurch Schalten des schaltbaren Klebstoffs von einem Aufbringzustand in einen Tragezustand,
   - Exponieren des schaltbaren Klebstoffs gegenüber einem zweiten Schaltaktivator, dadurch Schalten des schaltbaren Klebstoffs von dem Tragezustand in einen Entfernzustand,
   - Entfernen der Klebstoffzusammensetzung von der Haut des Anwenders,

   wobei der erste Schaltaktivator Licht ist und wobei der zweite Schaltaktivator Licht ist.

2. Verfahren gemäß einem der vorstehenden Ansprüche, wobei die beiden verschiedenen Photoinitiatoren unabhängig ausgewählt sind aus der Gruppe bestehend aus α-Hydroxyketon, Benzophenon, Benzophenonderivaten, Benzophenon/α-Hydroxyketon, Phenylglyoxylat, Benzyldimethylketal, Aminoketon, Acylphosphinderivaten, Monoacylphosphin (MAPO), MAPO/α-Hydroxyketon, Bisacylphosphin (BAPO), BAPO-Dispersion, BAPO/α-Hydroxyketon, Phosphinoxid, Metallocen, Ioniumsalz, Thioxanthonderivaten, Gemisch von Triarylsulfoniumhexafluorphosphatsalzen in Propylencarbonat, Gemisch von Triarylsulfoniumhexafluorantimonatsalzen in Propylencarbonat, Amphorchinonderivaten, Benzilderivaten, Anthrachinonderivaten, Benzoinetherderivaten, Polysilanen, 2-Benzyl-2-(dimethylamino)-4'-morpholinobutyrophenon, 2-Methyl-4'-(methylthio)-2-morpholinopropiophenon, (Benzol)tricarbonylchrom, (Cumen)cyclopentadienyleisen(II)-hexafluorphosphat, Dibenzosuberenon, Ferrocen, Methylbenzoylformiat

und Gemischen davon.

3. Verfahren gemäß einem der vorstehenden Ansprüche, wobei der Schaltaktivator Licht ausgewählt aus der Gruppe bestehend aus sichtbarem Licht, UV-Licht und Gemischen davon ist.

4. Verfahren gemäß einem der vorstehenden Ansprüche, wobei die Klebstoffzusammensetzung ein Haftplymer ausgewählt aus der Gruppe bestehend aus Acrylatpolymeren, Acrylatcopolymeren und Polyurethanpolymeren umfasst.

5. Verfahren gemäß einem der vorstehenden Ansprüche, wobei die Klebstoffzusammensetzung in dem Aufbringzustand eine komplexe Viskosität |η*| von unter 0,4 MPa.s, wie hierin beschrieben gemessen, aufweist.

6. Verfahren gemäß einem der vorstehenden Ansprüche, wobei die Klebstoffzusammensetzung in dem Tragezustand eine höhere komplexe Viskosität, wie hierin beschrieben gemessen, als in dem Aufbringzustand aufweist.

7. Verfahren gemäß einem der vorstehenden Ansprüche, wobei die Klebstoffzusammensetzung in dem Tragezustand eine Abziehkraft von größer als 1 N/25 mm, wie hierin beschrieben gemessen, aufweist.

8. Verfahren gemäß einem der vorstehenden Ansprüche, wobei die Klebstoffzusammensetzung in dem Entfernzustand eine niedrigere Abziehkraft, wie hierin beschrieben gemessen, als in dem Tragezustand aufweist.

9. Verfahren gemäß einem der vorstehenden Ansprüche, wobei die Klebstoffzusammensetzung ferner wasserabsorbierendes Material umfasst.

10. Verfahren gemäß einem der vorstehenden Ansprüche, wobei die Klebstoffzusammensetzung ferner eine oder mehrere zusätzliche Komponenten ausgewählt aus der Gruppe bestehend aus Weichmachern, Ölen, Klebrigmachern, Füllstoffen und Viskositätsmodifikatoren umfasst.

11. Verfahren gemäß einem der vorstehenden Ansprüche, wobei die Klebstoffzusammensetzung in der Form einer Haftscheibe bereitgestellt wird, die die Klebstoffzusammensetzung, eine Trägerschicht und eine Abziehschicht umfasst.

12. Verfahren gemäß einem der vorstehenden Ansprüche, wobei die Klebstoffzusammensetzung in der Form einer Ostomievorrichtung, die die Klebstoffzusammensetzung umfasst, bereitgestellt wird.

## Revendications

1. Méthode non thérapeutique d'application d'une composition adhésive commutable à la lumière sur la peau d'un utilisateur et de retrait de la composition adhésive commutable à la lumière de la surface de la peau, dans laquelle la composition adhésive commutable à la lumière comprend au moins deux photoinitiateurs différents de spectres d'absorption différents, la méthode comprenant les étapes consistant à

- se munir d'une composition adhésive comprenant un adhésif commutable,
- appliquer la composition adhésive à la peau de l'utilisateur,
- exposer l'adhésif commutable à un premier activateur de commutation, ce qui permet de commuter l'adhésif commutable d'un état d'application à un état de port,
- exposer l'adhésif commutable à un deuxième activateur de commutation, ce qui permet de commuter l'adhésif commutable de l'état de port à un état de retrait,
- retirer la composition adhésive de la peau de l'utilisateur,

dans laquelle le premier activateur de commutation est la lumière et dans laquelle le deuxième activateur de commutation est la lumière.

2. Procédé selon l'une quelconque des revendications précédentes, dans lequel les deux photoinitiateurs différents sont indépendamment choisis dans le groupe constitué par $\alpha$-hydroxycétone, benzophénone, dérivés de benzophénone, benzophénone/$\alpha$-hydroxycétone, glyoxylate de phényle, benzyldiméthylcétal, aminocétone, dérivés d'acylphosphine, monoacylphosphine (MAPO), MAPO/$\alpha$-hydroxycétone, bisacylphosphine (BAPO), dispersion de BAPO, BAPO/$\alpha$-hydroxycétone, oxyde de phosphine, métallocène, sel d'ionium, dérivés de thioxanthone, mélange de sels

d'hexafluorophosphate de triarylsulfonium dans le carbonate de propylène, mélange de sels d'hexafluoroantimonate de triarylsulfonium dans le carbonate de propylène, dérivés d'amphorquinone, dérivés benzyliques, dérivés d'anthraquinone, dérivés d'éther de benzoïne, polysilanes, 2-benzyl-2-(diméthylamino)-4'-morpholinobutyrophénone, 2-méthyl-4'-(méthylthio)-2-morpholinopropiophénone, (benzène)tricarbonylchrome, hexafluorophophate de (cumène)cyclopentadiénylfer(II), dibenzosubérénone, ferrocène, formiate de méthylbenzoyle, et leurs mélanges.

**3.** Méthode selon l'une quelconque des revendications précédentes, dans laquelle l'activateur de commutation est une lumière choisie dans le groupe constitué par la lumière visible, la lumière UV et leurs mélanges.

**4.** Méthode selon l'une quelconque des revendications précédentes, dans laquelle la composition adhésive comprend un polymère adhésif choisi dans le groupe constitué par les polymères d'acrylate, les copolymères d'acrylate et les polymères de polyuréthane.

**5.** Méthode selon l'une quelconque des revendications précédentes, dans laquelle la composition adhésive dans l'état d'application présente une viscosité complexe $|\eta^*|$ inférieure à 0,4 MPa.s, mesurée comme décrit dans la présente invention.

**6.** Méthode selon l'une quelconque des revendications précédentes, dans laquelle la composition adhésive dans l'état de port présente une viscosité complexe supérieure à celle de l'état d'application, mesurée comme décrit dans la présente invention.

**7.** Méthode selon l'une quelconque des revendications précédentes, dans laquelle la composition adhésive dans l'état de port présente une résistance au décollement supérieure à 1 N/25 mm, mesurée comme décrit dans la présente invention.

**8.** Méthode selon l'une quelconque des revendications précédentes, dans laquelle la composition adhésive présente une résistance au décollement dans l'état de retrait inférieure à celle de l'état de port, mesurée comme décrit dans la présente invention.

**9.** Méthode selon l'une quelconque des revendications précédentes, dans laquelle la composition adhésive comprend en outre une matière hydroabsorbante.

**10.** Méthode selon l'une quelconque des revendications précédentes, dans laquelle la composition adhésive comprend en outre un ou plusieurs composants supplémentaires choisis dans le groupe constitué par les plastifiants, les huiles, les tackifiants, les charges et les modificateurs rhéologiques.

**11.** Méthode selon l'une quelconque des revendications précédentes, dans laquelle la composition adhésive se présente sous la forme d'une plaquette adhésive comprenant la composition adhésive, une couche de support et une doublure de décollement.

**12.** Méthode selon l'une quelconque des revendications précédentes, dans laquelle la composition adhésive se présente sous la forme d'un dispositif de stomie comprenant la composition adhésive.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012048128 A
- WO 9013420 A
- WO 2014093246 A